# EUROPEAN PATENT APPLICATION

(11) **EP 4 252 531 A1**
(43) Date of publication of application: **04.10.2023**
(21) Application number: 21897646.2
(22) Date of filing: 02.11.2021
(51) Int. Cl.: A01K 67/033

(54) **REARING DEVICE**

(30) Priority: 26.11.2020 JP 2020195731
(71) Applicant: JTEKT Corporation, Kariya-shi, Aichi-ken, 448-8652 (JP); Tokushima University, Tokushima-shi, Tokushima 770-8501 (JP)
(72) Inventor: MURATA Yasuhiro, Kariya-shi, Aichi 448-8652 (JP); TAKAZATO Akihiro, Kariya-shi, Aichi 448-8652 (JP); KAWAI Shigekazu, Kariya-shi, Aichi 448-8652 (JP); MIURA Nozomu, Kariya-shi, Aichi 448-8652 (JP); WATANABE Takahito, Tokushima-shi, Tokushima 770-8501 (JP); MITO Taro, Tokushima-shi, Tokushima 770-8501 (JP)
(74) Representative: TBK
(86) International application number: PCT/JP2021/040441
(87) International publication number: WO 2022/113670

(57) **Abstract**

There is provided a rearing apparatus (1) including a hatching area (A) that holds eggs of organisms to be reared and accommodates the organisms hatched from the eggs, and a rearing area (B) that is defined in an area larger than the hatching area (A) at a position different from the hatching area (A) and in which the organisms are reared. The present disclosure provides a rearing apparatus that can implement high-density and large-scale rearing by effectively utilizing areas.

## Description

### TECHNICAL FIELD

The present disclosure relates to a rearing apparatus.

### BACKGROUND ART

JPH10-191834A describes a rearing apparatus for crickets. In the rearing apparatus, a rearing case is provided with a container in which absorbent cotton containing water is placed to supply water to the crickets, and spawning, hatching, and rearing are all performed in the rearing case.

WO2016-153338A1 describes a facility for rearing insects. The facility includes a spawning container, a baby larvae container, a pupating container, and the like, and includes a belt conveyor that conveys the containers.

In the rearing apparatus described in JPH10-191834A, the rearing case has a size (volume) capable of appropriately rearing grown crickets since spawning to rearing are performed in one rearing case.

For this reason, the proportion (density) of the crickets relative to the volume of the rearing case immediately after hatching is fairly small. That is, the space of the rearing case cannot be effectively utilized immediately after hatching.

In WO2016-153338A1, containers for spawning, hatching, baby larvae rearing, mature larvae rearing, and the like are separate containers, and a structure for moving the containers is fairly complicated and large in size.

### SUMMARY OF INVENTION

The present disclosure provides a rearing apparatus that can implement high-density and large-scale rearing by effectively utilizing areas.

According to an aspect of the present disclosure, a rearing apparatus includes: a hatching area that holds eggs of organisms to be reared and accommodates the organisms hatched from the eggs; and a rearing area that is defined in an area larger than the hatching area at a position different from the hatching area to rear the organisms.

The size of eggs and organisms immediately after hatching is different from the size of grown organisms. Therefore, the rearing area is larger than the hatching area. That is, each of the hatching area and the rearing area can have a size corresponding to the size of organisms to be reared. Therefore, the density of eggs and organisms in the hatching area can be increased, and the density of organisms in the rearing area can also be increased. In this way, the rearing can be performed at high density in each of the hatching area and the rearing area.

As an example of the rearing apparatus, it is preferable that the rearing area be arranged adjacently to the hatching area, and that rearing area be configured to move the organisms hatched in the hatching area out of the hatching area in a state where the hatching area is opened to the rearing area. In this case, the organisms hatched in the hatching area can automatically move from the hatching area to the rearing area without the movement by an operator or the movement by an actuator. That is, large-scale and high-density rearing can be implemented.

### BRIEF DESCRIPTION OF DRAWINGS

Fig. 1 is a schematic view of rearing areas in a rearing apparatus;
Fig. 2 is a front view of the rearing apparatus;
Fig. 3 is a top view of the rearing apparatus (that is, a view seen from above in Fig. 2);
Fig. 4 is a right side view of the rearing apparatus (that is, a view seen from right of Fig. 2);
Fig. 5 is a cross-sectional view taken along a line V-V in Fig. 4;
Fig. 6 is a cross-sectional view taken along a line VI-VI in Fig. 5;
Fig. 7 is a cross-sectional view taken along a line VII-VII in Fig. 5;
Fig. 8 is a cross-sectional view of the rearing apparatus at the time of harvest (that is, a cross-sectional view corresponding to Fig. 5);
Fig. 9 is a cross-sectional view taken along a line IX-IX in Fig. 8;
Fig. 10 is a functional block diagram related to a control device configuring the rearing apparatus; and
Fig. 11 is a flowchart related to temperature and humidity control by the control device.

### DESCRIPTION OF EMBODIMENTS

### <1. Organisms To Be Reared>

Organisms to be reared in a rearing apparatus are, for example, small organisms such as arthropods. The organisms to be reared are used for, for example, food, feed, and research. Examples of insects among the organisms to be reared include crickets, locusts, and grasshoppers. In particular, as a preferable example, the organisms to be reared are arthropods with incomplete metamorphosis in which larvae directly metamorphose into adult insects. Further, larvae in incomplete metamorphosis are preferable as the organisms. Among the insects, larvae of crickets and locusts belonging to the order Orthoptera will be described as a preferable example of the organisms to be reared in the present embodiment. Adult insects (emerged incomplete metamorphosis arthropods) may also be used as the organisms to be reared.

A rearing period of the organisms to be reared is defined as, for example, a period starting from eggs as an early stage to the last stage immediately before becoming adult insects. For example, larvae of crickets hatched from eggs grow by repeating molting plural times (seventh to eighth instars), and then emerge to become adult insects. When crickets are to be reared, for example, the crickets are reared in the rearing apparatus from eggs until larvae after plural times of molting.

### <2. Overview of Rearing Areas in Rearing Apparatus 1>

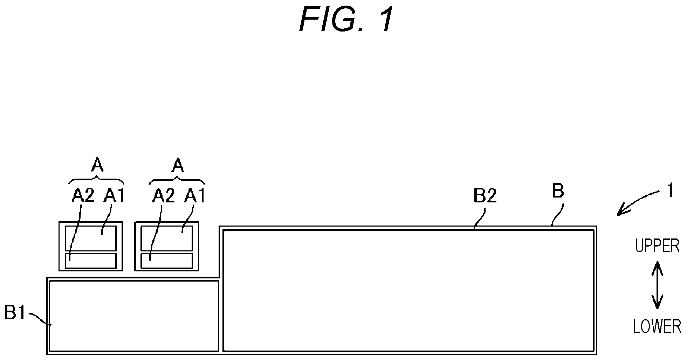

An overview of rearing areas in a rearing apparatus 1 will be described with reference to Fig. 1. As described above, organisms to be reared are reared in the rearing apparatus 1 in a rearing period starting from eggs as the early stage to the last stage before becoming adult insects.

As shown in Fig. 1, the rearing apparatus 1 includes hatching areas A that hold eggs of the organisms to be reared and accommodate larvae of the organisms hatched from the eggs, and a rearing area B that is defined at a position different from the hatching areas A and in which the larvae of the organisms are reared. In the present embodiment, the rearing apparatus 1 includes two hatching areas A and one rearing area B for rearing organisms hatched in the two hatching areas A.

The hatching area A includes an egg holding area A1 that holds eggs of organisms, and a baby larvae rearing area A2 that is adjacent to the egg holding area A1 and in which baby larvae of the organisms are reared. In the present embodiment, the baby larvae rearing area A2 is located below the egg holding area A1 in the hatching area A. When the larvae hatched in the egg holding area A1 fall from the egg holding area A1, the larvae can move to the baby larvae rearing area A2. That is, a worker does not carry the larvae, and it is not necessary to use an actuator such as a belt conveyor. The egg holding area A1 and the baby larvae rearing area A2 may also be adjacent to each other in a horizontal direction.

Here, the hatching area A is implemented by an enclosed area, and unless the hatching area A is opened by an external force, the hatched larvae cannot get out of the hatching area A. In the present embodiment, for example, first instar larvae to second to fourth instar larvae of crickets can be reared in the baby larvae rearing area A2. The size of the baby larvae rearing area A2 may correspond to the size of baby larvae to be reared since the crickets grow larger upon each molting. For example, the baby larvae rearing area A2 may be larger when rearing up to fourth instar larvae than when rearing up to second instar larvae.

The rearing area B is arranged adjacently to the hatching area A. The rearing area B may be arranged adjacently to the hatching area A in a vertical direction or the horizontal direction, for example. The rearing area B is defined in an area larger than the hatching area A. In the rearing area B, mature larvae after moved from the hatching area A, particularly from the baby larvae rearing area A2, are reared. Here, the mature larvae are larger than the baby larvae. Therefore, the rearing area B is larger than the hatching area A, so that the rearing area B can be an area suitable for growth of the mature larvae.

In a state where the hatching area A is opened to the rearing area B, the larvae hatched in the hatching area A can move out of the hatching area. For example, in the present embodiment, at least a part of the rearing area B is below and adjacent to the hatching area A. In this case, when the larvae hatched in the hatching area A fall from the hatching area A in a state in which a lower surface of the hatching area A is opened to the rearing area B, the larvae can move out of the hatching area A.

The rearing area B is provided with, on a lower surface thereof, a floor surface so that the organisms to be reared can move. Therefore, the organisms falling from the hatching area A fall on the floor surface, and can freely move along the floor surface in the rearing area B.

The rearing area B may include a falling area B1 (that is, an organism moving area) and a perching member area B2. The falling area B1 is, for example, an area that is located below the hatching area A and to which the larvae fall from the hatching area A. That is, the falling area B1 is located below the baby larvae rearing area A2 of the hatching area A, and the baby larvae fall from the baby larvae rearing area A2 when a lower side of the hatching area A is opened.

The perching member area B2 in the rearing area B is adjacent to the falling area B1 in the horizontal direction, and includes a perching member on which larvae can perch. The perching member area B2 can be used as a hiding place for the larvae, in which an environment suitable for rearing is provided. That is, the perching member area B2 functions as a main area for the growth of the mature larvae. Therefore, the perching member area B2 is defined in an area larger than the hatching area A. The larvae present in the falling area B1 can move to the perching member area B2 by its own moving ability.

As described above, the hatched larvae move by falling from the hatching area A to the rearing area B. That is, the hatching area A is disposed at a position higher than the rearing area B. Therefore, the larvae moved to the rearing area B cannot return to the hatching area A. That is, the larvae moved to the rearing area B grow in the rearing area B. Then, another hatching can be implemented by installing new eggs in the hatching area A.

### <3. Effects of Areas A and B>

Effects of providing the hatching area A and the rearing area B in the rearing apparatus 1 will be described. The size of eggs and larvae immediately after hatching is different from the size of grown larvae. Therefore, the rearing area B is larger than the hatching area A. That is, each of the hatching area A and the rearing area B can have a size corresponding to the size of organisms to be reared. Therefore, the density of eggs and organisms in the hatching area A can be increased, and the density of organisms in the rearing area B can also be increased. In this way, rearing can be performed at high density in each of the hatching area A and the rearing area B.

In the rearing apparatus 1, the rearing area B is adjacent to the hatching area A, and the larvae hatched in the hatching area A can move out of the hatching area A in a state where the hatching area A is opened to the rearing area B. In this case, the larvae hatched in the hatching area A can automatically move from the hatching area A to the rearing area B without the movement by the worker or the movement by the actuator. That is, large-scale and high-density rearing can be implemented.

### <4. Specific Configuration of Rearing Apparatus 1>

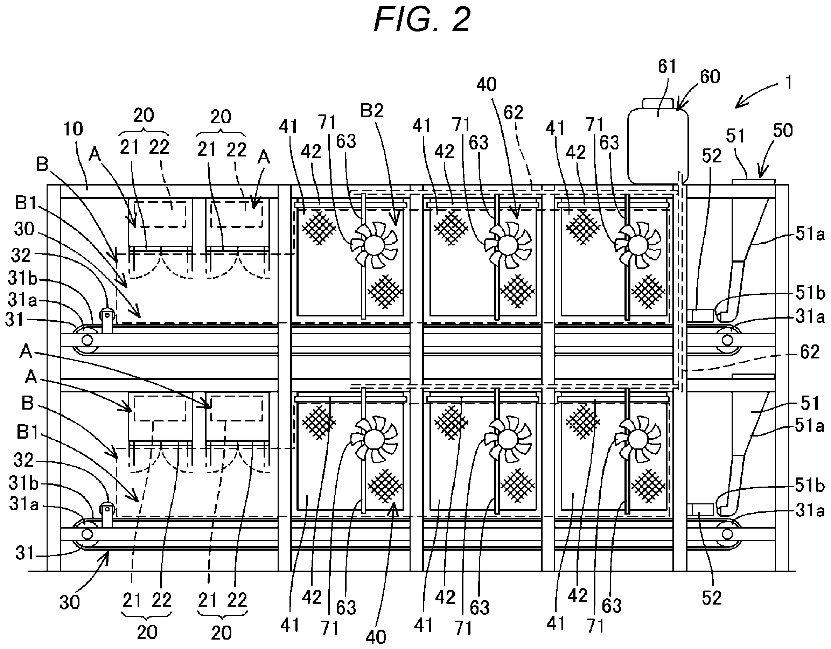

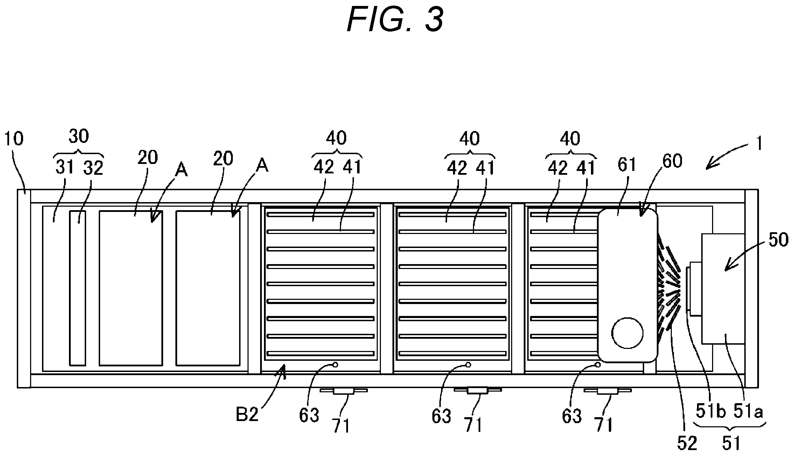

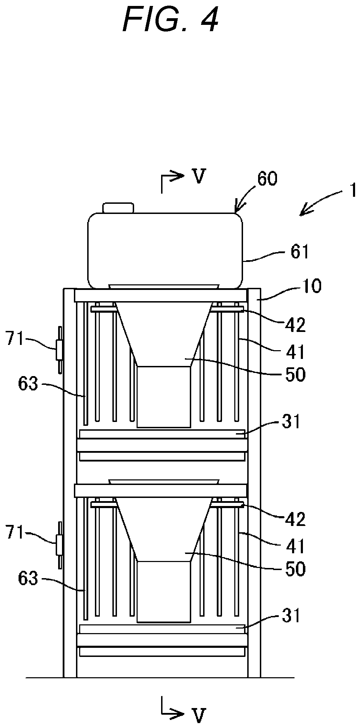

Next, a specific configuration of the rearing apparatus 1 will be described with reference to Figs. 2 to 7. As shown in Fig. 2, the rearing apparatus 1 includes a frame 10, hatching cases 20, conveyor units 30, rearing area facilities 40, feeding devices 50, a water supply device 60, and humidity control blowers 71.

The frame 10 is fixed to a ground contact surface, and two hatching areas A and one rearing area B shown in Fig. 1 can be arranged in two layers. In the present embodiment, the rearing apparatus 1 has two layers, and may also have one layer or three or more layers. The frame 10 has a rectangular parallelepiped shape in which the horizontal direction is defined as a longitudinal direction of the frame 10 for each layer.

The hatching case 20 configures the hatching area A in Fig. 1. Two hatching cases 20 are fixed to an upper side of one end (left end in Fig. 2) of the frame 10 in the longitudinal direction in each layer. The hatching case 20 has a rectangular parallelepiped shape, and includes a door 21 whose lower surface can be opened. When the door 21 is opened, the organisms to be reared present in the hatching case 20 fall.

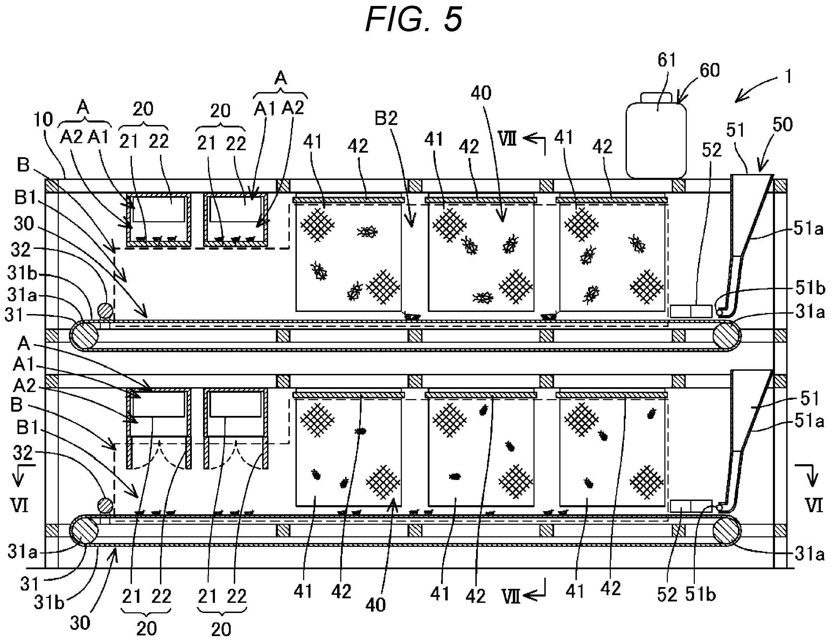

The hatching case 20 further includes an egg holding member 22 therein. As shown in Fig. 5, the egg holding member 22 is disposed on an upper side in the hatching case 20. The egg holding member 22 holds plural eggs of organisms to be reared, and is a place where the eggs can hatch. An arrangement area of the egg holding member 22 configures the egg holding area A1 shown in Fig. 1.

In the hatching case 20, the baby larvae rearing area A2 is defined below the egg holding member 22. Therefore, organisms hatched in the egg holding member 22 move to the baby larvae rearing area A2. The organisms present in the baby larvae rearing area A2 fall below the hatching case 20 when the door 21 is opened. The baby larvae rearing area A2 may be provided with a member serving as a hiding place.

The conveyor unit 30 is disposed at a bottom portion of each layer of the frame 10. The conveyor unit 30 includes a belt conveyor 31 and a compression discharge roller 32. An upper surface of the belt conveyor 31 configures a bottom surface of the rearing area B. In the present embodiment, a configuration including the belt conveyor 31 is exemplified, and a bottom surface that is not movable may also be provided instead of the belt conveyor 31.

The belt conveyor 31 includes a pair of support rollers 31a and an endless belt 31b installed over the pair of support rollers 31a. When the support rollers 31a are driven, the belt 31b can continuously move. Here, a conveying direction of the belt conveyor 31 means a moving direction of an upper surface of the belt 31b. In Fig. 2, the conveying direction of the belt conveyor 31 is a direction from right to left.

The compression discharge roller 32 configuring the conveyor unit 30 is disposed on a downstream side of the belt conveyor 31, and is in contact with the upper surface of the belt 31b. The compression discharge roller 32 compresses and discharges empty shells, feces, and the like present on the belt 31b. However, the compression discharge roller 32 may be disposed with a slight gap from the upper surface of the belt 31b. When organisms to be reared present on the belt 31b approach the compression discharge roller 32 in a state in which the belt conveyor 31 is driven, the organisms escape before being compressed.

The rearing area facility 40 configures the rearing area B shown in Fig. 1. The rearing area facility 40 includes the falling area B1 (that is, an organism movement area) located below the hatching case 20 and the perching member area B2 located on an upstream side of the belt conveyor 31 relative to the falling area B1. As shown in Fig. 2, the falling area B1 is provided with no member, and does not hinder the organisms in the hatching case 20 from falling from the hatching case 20 onto the upper surface of the belt conveyor 31.

As shown in Figs. 2 to 7, the perching member area B2 of the rearing area facility 40 is provided with perching members 41. The perching member 41 is fixed to the frame 10 while extending in the vertical direction in the perching member area B2, so that the organisms to be reared can perch. The perching member 41 further functions as a hiding place for the organisms. A lower end of the perching member 41 is disposed in a position slightly away from the upper surface of the belt 3 1b of the belt conveyor 31. When the belt 3 1b moves, the belt 31b does not come into contact with the perching member 41.

The perching member 41 includes, for example, plural plate portions arranged to face each other in the horizontal direction. In the present embodiment, each of the plate portions has a flat plate shape, and may also have a wave shape. In the present embodiment, each of the plural plate portions configuring the perching member 41 extends in the conveying direction of the belt conveyor 31. That is, the plural plate portions configuring the perching member 41 are arranged to face each other in a left-right direction when the conveying direction is front. The plural plates configuring the perching member 41 may be arranged to face each other in the conveying direction, or may be arranged to face each other in a direction inclined relative to the conveying direction. In the present embodiment, the perching member 41 is configured from three units in the conveying direction of the belt conveyor 31. The number of units of the perching member 41 may be freely selected.

The perching member 41 is formed of metal such as iron or aluminum, resin, wood, paper, and the like. The perching member 41 has plural pores over the entire surface to serve as scaffolds for organisms such as insects. For example, the perching member 41 is formed of a punching metal, a wire mesh, or the like. The perching member 41 has a thickness of, for example, 1 to 5 [mm], and has pores with a diameter of about 1 [mm] and a pitch of about 2 [mm]. To function as a hiding place, an interval between adjacent plate portions configuring the perching member 41 is, for example, 10 to 30 [mm].

As shown in Figs. 2 to 7, the rearing area facility 40 further includes separation members 42. The separation member 42 is a member that separates organisms perched on the perching member 41 from the perching member 41. The separation member 42 is formed of metal such as iron and aluminum, resin, wood, and the like. The separation member 42 has a slit through which the perching member 41 can be inserted, and is movable in the vertical direction along the perching member 41. In the present embodiment, the separation member 42 has a plate shape parallel to the horizontal direction, and has plural slits through which the plural plate portions configuring the perching member 41 can be inserted.

The separation member 42 moves downward from a state of being located at an upper end of the perching member 41, so that the organisms perched on the perching member 41 can fall onto the upper surface of the belt 31b. When the separation member 42 moves to a lower end of the perching member 41, most of the organisms move to the upper surface of the belt 31b. Since the separation member 42 has a plate shape parallel to the horizontal direction, the organisms cannot move upward beyond the separation member 42.

The vertical movement of the separation member 42 is manually operated by a worker, for example. When the worker manually operates the separation member 42, for example, the worker can vertically move the separation member 42 in conjunction with rotation of a manual handle (not shown) by rotationally operating the manual handle. In addition to the manual handle, an operation rod (not shown) coupled to the separation member 42 may be vertically moved to vertically move the separation member 42.

The feeding device 50 is a device for supplying feed to the organisms reared in the rearing area B. The feeding device 50 is disposed on the upstream side in the conveying direction of the belt conveyor 31 in the frame 10. The feeding device 50 includes a feeding body 51 that supplies feed to the upper surface of the belt conveyor 31, and a diffusion mechanism 52.

The feeding body 51 includes a hopper 51a that holds feed, and a rotary brush 51b that switches between a feeding state and a feeding stop state of feed from an outlet of the hopper 51a. The hopper 51a is formed to become smaller in a horizontal area from an upper end opening downward. The rotary brush 51b is provided at a lower end opening of the hopper 51a, supplies feed to the upper surface of the belt conveyor 31 during rotation, and stops the supply of feed when the rotation is stopped. The feeding body 51 has a feed supply port located at a center of the belt conveyor 31 in a width direction.

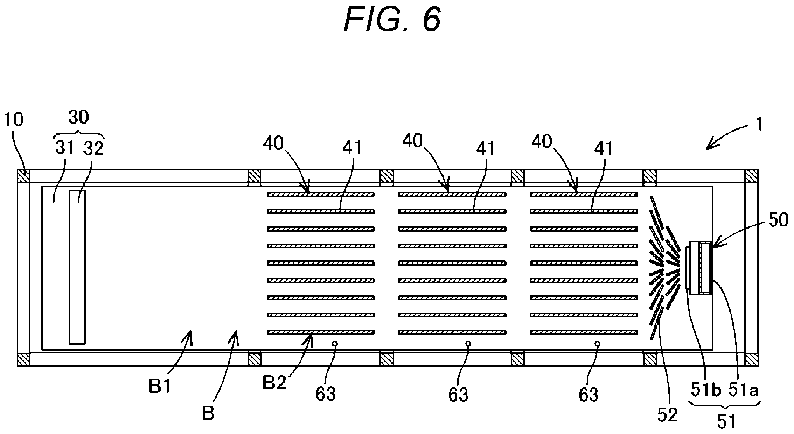

As shown in Fig. 6, the diffusion mechanism 52 diffuses the feed supplied from the feeding body 51 in the width direction of the belt conveyor 31. In the present embodiment, the diffusion mechanism 52 includes plural diffusion plates. As described above, the feed supply port of the feeding body 51 is located at the center of the belt conveyor 31 in the width direction. Therefore, the diffusion plates configuring the diffusion mechanism 52 are inclined outward in the width direction from the center in the width direction and from the upstream side to the downstream side of the belt conveyor 31 in the conveying direction.

Therefore, the diffusion plates configuring the diffusion mechanism 52 can diffuse the feed at the center of the belt conveyor 31 in the width direction over the entire width direction of the belt conveyor 31. Then, in the area in which the perching member 41 is located, the feed is diffused over the entire width direction of the belt conveyor 31. Although not shown, the diffusion plates configuring the diffusion mechanism 52 are fixed to the frame 10. In addition to the diffusion plates, a rotary brush for diffusion or the like may be used as the diffusion mechanism 52. The rotary brush for diffusion can diffuse the feed, for example, by rotating about an axis inclined relative to the conveying direction of the belt conveyor 31.

The water supply device 60 supplies water to the organisms perched on the perching member 41. The water supply device 60 includes a water tank 61, pipes 62, and water containing members 63. The water tank 61 is fixed to an uppermost stage of the frame 10. The pipe 62 is fixed along the frame 10, and is provided in a path from the water tank 61 to the perching member 41.

The water containing member 63 is formed of a water containing material. The water containing material is preferably, for example, a material on which organisms can perch while containing water. An example of the water containing material includes a polymer absorbent material. The water containing member 63 has, for example, a string shape or a band shape, and extends downward from a pipe. A lower end of the water containing member 63 extending downward is located in the vicinity of the upper surface of the belt conveyor 31.

Therefore, the water containing member 63 can come into contact with organisms present on the upper surface of the belt conveyor 31. The water containing member 63 can supply water to the organisms. In the present embodiment, the water containing member 63 is located in the vicinity of a center of the perching member 41 in the conveying direction of the belt conveyor 31, and is located further outward than the plate portion at one end of the belt conveyor 31 in the width direction among the plural plate portions configuring the perching member 41.

The humidity control blower 71 is fixed to the frame 10, and is arranged to face the plate portions configuring the perching member 41 in the width direction of the belt conveyor 31. Further, the humidity control blower 71 is arranged to face the water containing member 63 in the width direction of the belt conveyor 31. That is, the water containing member 63 is located between the humidity control blower 71 and the perching member 41.

The humidity control blower 71 humidifies the inside of the rearing area B using the moisture of the water containing member 63 by blowing air toward the perching member 41 and the water containing member 63 (by blowing air in a forward direction). Conversely, the humidity control blower 71 can suction air from the inside of the rearing area B by blowing air toward a side opposite to the perching member 41 and the water containing member 63, that is, toward outside (in other words, by blowing air in a reverse direction). In this case, since the water containing member 63 is located downstream of the perching member 41 in the blowing direction, the rearing area B is not humidified.

### <5. Operation of Rearing Apparatus 1>

Operation of the rearing apparatus 1 will be described with reference to Figs. 2, 5, and 7 to 9. As shown in Fig. 2, in an initial state of the rearing apparatus 1, the door 21 of the hatching case 20 is closed, and the separation member 42 in the rearing area facility 40 is located in the vicinity of the upper end of the perching member 41.

First, the worker places eggs to be reared in the egg holding member 22 (that is, the egg holding area A1) of the hatching case 20. When time elapses, the eggs held by the egg holding member 22 hatch and move to the baby larvae rearing area A2 of the hatching case 20. At this time, since the door 21 of the hatching case 20 is in a closed state, baby larvae grow in the baby larvae rearing area A2.

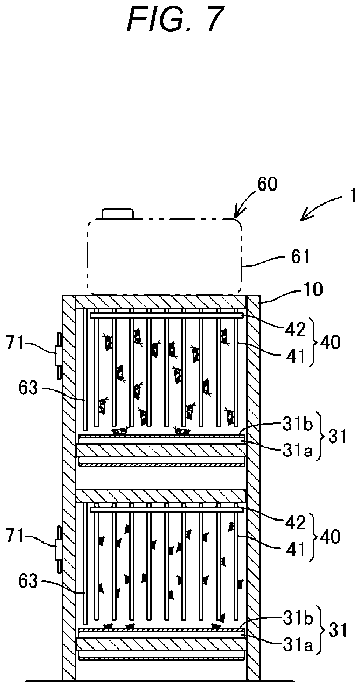

Subsequently, when time elapses, the door 21 of the hatching case 20 is opened as shown in a lower layer of Fig. 5. Then, the baby larvae present in the baby larvae rearing area A2 fall, move to the falling area B1 of the rearing area B, and reach the upper surface of the belt conveyor 31 in the falling area B1. The baby larvae moved to the falling area B1 move to the perching member area B2 along the upper surface of the belt conveyor 31 by its own strength. The baby larvae moved to the perching member area B2 climb over the perching member 41 and grow with the perching member 41 as a hiding place, as shown in lower layers of Figs. 5 and 7.

Here, when the larvae are present in the rearing area B, the feeding device 50 periodically supplies feed to the upper surface of the belt conveyor 31. At this time, by driving the rotary brush 5 1b of the feeding body and driving the belt conveyor 31 at the same time, the feed is supplied to the entire upper surface of the belt conveyor 31 in the perching member area B2 in the conveying direction and the width direction.

When the larvae are present in the rearing area B, water is contained in the water containing member 63 from the water tank 61 via the pipe 62 periodically or constantly. The larvae present in the perching member 41 once descend from the perching member 41 onto the upper surface of the belt conveyor 31, they move to the position of the water containing member 63, and receive water at the water containing member 63.

Further, the larvae present in the rearing area B grow and repeat molting, so that empty shells and feces accumulate on the upper surface of the belt conveyor 31. Therefore, the upper surface of the belt conveyor 31 is cleaned by periodically driving the belt conveyor 31.

After the baby larvae move from the hatching case 20 to the rearing area B, nothing is present in the hatching case 20. Therefore, as shown in upper layers of Figs. 5 and 7, the worker again places eggs to be reared in the egg holding member 22 (egg holding area A1) of the hatching case 20. Then, as described above, the eggs hatch and baby larvae are reared in the hatching case 20.

That is, in this state, hatching and rearing of the baby larvae are performed in the hatching case 20 (hatching area A) of the rearing apparatus 1, and rearing of the mature larvae is performed in the rearing area B. In this way, two generations of rearing are performed in parallel.

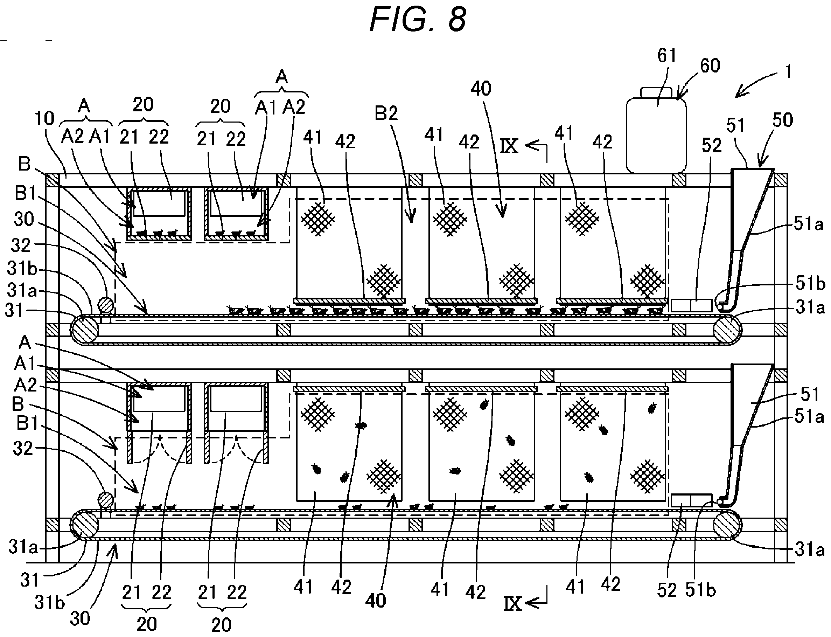

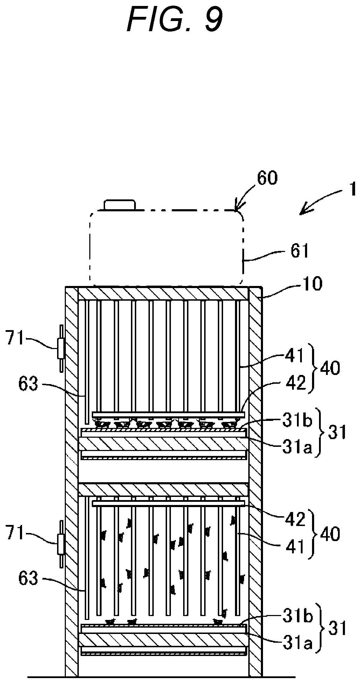

Subsequently, when the baby larvae grow sufficiently in the rearing area B, the separation member 42 is moved downward as shown in upper layers of Figs. 8 and 9. This operation may be performed by an automatic operation by an actuator or by a manual operation by the worker. By this operation, grown organisms perched on the perching member 41 are unloaded onto the upper surface of the belt conveyor 31.

Subsequently, the grown organisms can be conveyed to the downstream side of the belt conveyor 31 by driving the belt conveyor 31. The worker harvests the conveyed organisms. At this time, the compression discharge roller 32 is moved upward to increase the gap between the compression discharge roller 32 and the belt conveyor 31, so that the grown organisms can pass through the compression discharge roller 32. Then, the worker can easily harvest the grown organisms at a downstream end of the belt conveyor 31.

When there is no organisms in the rearing area B, as shown in the lower layer of Fig. 5, the door 21 in the hatching case 20 is opened again. At this time, the baby larvae can move to the rearing area B and grow in the rearing area B. Then, the above operation is repeated. Therefore, rearing can be performed at high density in each of the hatching area A and the rearing area B.

### <6. Temperature and Humidity Management of Rearing Apparatus 1>

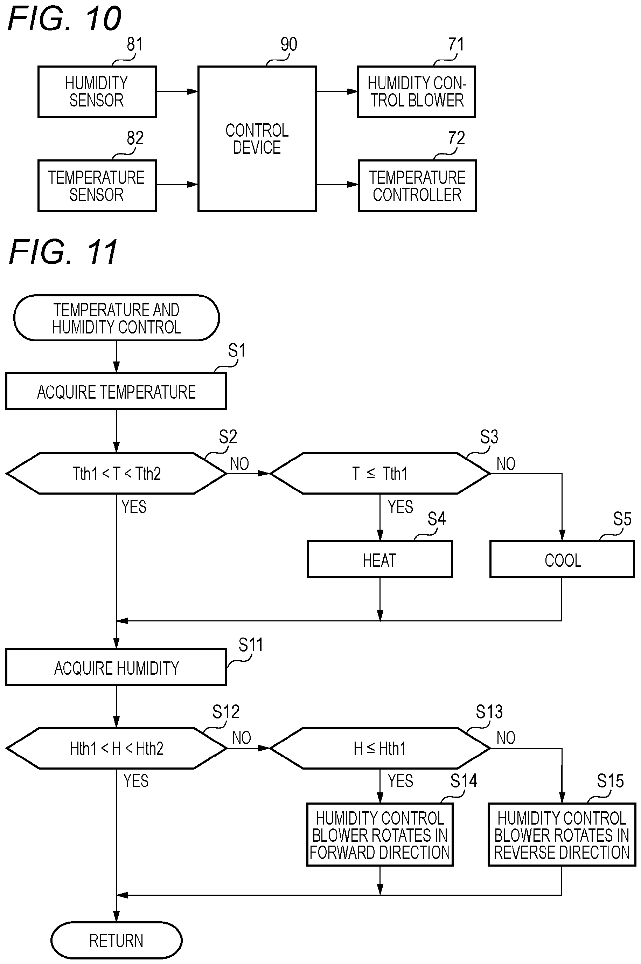

Next, temperature and humidity management in the rearing apparatus 1 will be described with reference to Figs. 10 and 11. In the middle of rearing organisms in the rearing area B of the rearing apparatus 1, management of temperature and humidity is fairly necessary. Any type of organisms have temperature and humidity suitable for the organisms. Therefore, temperature and humidity management in the rearing area B will be described.

As shown in Fig. 10, the rearing apparatus 1 includes the humidity control blower 71, a temperature controller 72, a humidity sensor 81, a temperature sensor 82, and a control device 90. As described above, the humidity control blower 71 is fixed to the frame 10, and faces the perching member 41 and the water containing member 63.

The temperature controller 72 may be fixed to the frame 10, and may also be, for example, a device that can control temperature of an entire room accommodating plural rearing apparatuses 1. The temperature controller 72 is a known air conditioner. The humidity sensor 81 and the temperature sensor 82 are disposed in the rearing area B and detect humidity and temperature in the rearing area B. The control device 90 controls the humidity control blower 71 based on the humidity detected by the humidity sensor 81. The control device 90 controls the temperature controller 72 based on the temperature detected by the temperature sensor 82.

A control method by the control device 90 will be described with reference to Fig. 11. The control device 90 acquires temperature T from the temperature sensor 82 (S1). When the acquired temperature T is higher than a lower limit threshold Tth1 and lower than an upper limit threshold Tth2 (S2: Yes), control of the temperature controller 72 maintains a current state.

When the acquired temperature T is equal to or lower than the lower limit threshold Tth1 (S2: No, S3: Yes), heating control of the temperature controller 72 is performed (S4). On the other hand, when the acquired temperature T is equal to or higher than the upper limit threshold Tth2 (S2: No, S3: No), cooling control of the temperature controller 72 is performed (S5). Therefore, the temperature controller 72 is controlled such that the acquired temperature T is higher than the lower limit threshold Tth1 and lower than the upper limit threshold Tth2.

Subsequently (S2: Yes, after S4 and S5), the control device 90 acquires humidity H from the humidity sensor 81 (S11). When the acquired humidity H is higher than a lower limit threshold Hth1 and lower than an upper limit threshold Hth2 (S12: Yes), the control device 90 stops the humidity control blower 71.

When the acquired humidity H is equal to or less than the lower limit threshold Hth1 (S12: No, S13: Yes), the control device 90 rotates the humidity control blower 71 in the forward direction to blow air toward the perching member 41 through the water containing member 63 (S14). That is, the perching member area B2 is humidified.

On the other hand, when the acquired humidity H is equal to or higher than the upper limit threshold Hth2 (S12: No, S13: No), the control device 90 rotates the humidity control blower 71 in the reverse direction to discharge humid air in the perching member area B2 to the outside (S15). At this time, air passed through the water containing member 63 flows to the side opposite to the perching member 41. Therefore, moisture in the water containing member 63 does not move toward the perching member 41, and dehumidification of the perching member area B2 is implemented.

Therefore, the humidity control blower 71 is controlled such that the acquired humidity H is higher than the lower limit threshold Hth1 and lower than the upper limit threshold Hth2. Then, the above processing is performed again. That is, the temperature and the humidity are appropriately managed in the perching member area B2, and a space comfortable for the organisms to be reared can be created.

As a method for dehumidification, the humidity control blower 71 is rotated in the reverse direction, and the present invention is not limited thereto. For example, a blower different from the humidity control blower 71 may be provided and blow air toward the perching member 41 without interposing the water containing member 63. In addition, a moving mechanism that moves the humidity control blower 71 may be provided, and air may be blown toward the perching member 41 after the humidity control blower 71 is moved to a position not facing the water containing member 63.

The present application is based on JP2020-195731A filed on November 26, 2020, and the contents thereof are incorporated herein as reference.

## Claims

1. A rearing apparatus comprising:
a hatching area that holds eggs of organisms to be reared and accommodates the organisms hatched from the eggs; and
a rearing area that is defined in an area larger than the hatching area at a position different from the hatching area to rear the organisms.

2. The rearing apparatus according to claim 1, wherein
the hatching area includes:
an egg holding area that holds the eggs of the organisms; and
a baby larvae rearing area that is arranged adjacently to the egg holding area to rear baby larvae of the organisms, and
the rearing area is configured to rear mature larvae of the organisms.

3. The rearing apparatus according to claim 1 or 2, wherein
the rearing area is arranged adjacently to the hatching area, and
the rearing area is configured to move the organisms hatched in the hatching area out of the hatching area in a state where the hatching area is opened to the rearing area.

4. The rearing apparatus according to claim 3, wherein
the rearing area is arranged adjacently below the hatching area, and
the rearing area is configured to cause the organisms hatched in the hatching area to fall from the hatching area to move the organisms out of the hatching area in a state where a lower surface of the hatching area is opened to the rearing area.

5. The rearing apparatus according to claim 4, wherein
the hatching area is arranged adjacently above the rearing area to prevent the organisms moved to the rearing area from returning to the hatching area.

6. The rearing apparatus according to claim 4 or 5, wherein
the rearing area includes:
a falling area to which the organisms fall from the hatching area, the falling area being located below the hatching area and; and
a perching member area that includes a perching member on which the organisms are capable of perching, the perching member area being arranged adjacently to the falling area in a horizontal direction.

7. The rearing apparatus according to any one of claims 1 to 6, further comprising:
a belt conveyor formed as a lower surface of the rearing area;
a feeding body that is disposed on an upstream side of the belt conveyor in a conveying direction and supplies feed to an upper surface of the belt conveyor; and
a diffusion mechanism configured to diffuse the feed supplied from the feeding body in a width direction of the belt conveyor.

8. The rearing apparatus according to any one of claims 1 to 7, wherein
the rearing area includes:
a water containing member that is formed of a water containing material and is contactable with the organisms to supply water to the organisms; and
a humidity control blower that is arranged to face the water containing member, the humidity control blower humidifying an inside of the rearing area using moisture of the water containing member by blowing air toward the water containing member.
